# EUROPEAN PATENT SPECIFICATION

(11) **EP 1 102 994 B1**
(45) Date of publication and mention of the grant of the patent: **02.01.2003**
(21) Application number: 99934969.9
(22) Date of filing: 23.07.1999
(51) Int. Cl.: G01N 35/00

(54) **AUTOMATED IMMUNOASSAY APPARATUS WITH FLEXIBLE PICK-UP ARM**
AUTOMATISCHES IMMUNOASSAY-GERÄT MIT FLEXIBLEM HEBEARM
APPAREIL DE DOSAGE IMMUNOLOGIQUE AUTOMATISE MUNI D'UN DOIGT DE RAMASSAGE

(30) Priority: 04.08.1998 GB 9816943; 04.08.1998 GB 9816944
(43) Date of publication of application: 30.05.2001
(73) Proprietor: Dynex Technologies Inc., Chantilly, Virginia 22021 (US)
(72) Inventor: BOTT, Jon, St. Peter Port, Guernsey (GB); FUSSELLIER, Andrew, Torteval, Guernsey (GB); BUNCE, Adrian, Worthing West Sussex BN13 1JZ (GB); LE PAGE, Paul, Castel Guernsey GY5 0PD (GB)
(74) Representative: Jeffrey, Philip Michael
(86) International application number: GB9902425
(87) International publication number: WO00008472

(56) References cited:
- EP-A- 0 441 755
- EP-A- 0 557 828
- EP-A- 0 945 728
- WO-A-94/14073
- DE-A- 3 805 808
- US-A- 3 909 201
- US-A- 3 912 456
- US-A- 5 439 649
- LITTLE J N ET AL: "RECENT ADVANCES IN ROBOTIC AUTOMATION OF MICROPLATE ASSAYS" LABORATORY AUTOMATION & INFORMATION MANAGEMENT, vol. 26, no. 2, 1 November 1994 (1994-11-01), pages 89-99, XP000476920 ISSN: 1381-141X

## Description

The present invention relates to automated immunoassay apparatus for carrying out diagnostic testing, and to particularly useful apparatus for carrying out Enzyme Linked ImmunoSorbent Assay ("ELISA") procedures.

A number of different testing techniques for biological products are known and these include latex consumable tests and Polymerase Chain Reaction ("PCR") tests.

Latex consumable tests are used for example in home pregnancy testing kits, and are fast, reasonably accurate but comparatively expensive.

PCR tests are used mainly in research environments. Custom-made equipment is usually required in order for the technique to be reliably reproduced by laboratory technicians. Such equipment is comparatively expensive and is not generally compatible with other manufacturers' equipment.

Immunoassay procedures are a preferred way of testing biological products. These procedures exploit the ability of antibodies produced by the body to recognise specific antigens (which may, for example, be associated with foreign bodies such as bacteria or viruses, or with other body products such as hormones). Once a specific antigen has been detected by an antibody this can be indicated as a positive sample preferably by using fluorescent or chemiluminescent markers or less preferably by using radioactive markers. Radioactive markers are less preferred due to environmental and safety concerns regarding their handling, storage and disposal.

ELISA is a particularly preferred form of immunoassay procedure wherein antibodies are linked to an insoluble carrier surface such as a sample vessel. The antibodies are used to capture any counterpart antigens which may be present in a sample solution. If antigens are present then these bond with the antibodies to form antigen-antibody complexes. Substances known as "enzyme conjugates" are then added to the sample. An enzyme conjugate contains an enzyme which covalently bonds with the antigen part of any antigen-antibody complexes which have been formed. Colourless reagents are then added to the sample which are broken down in the presence of the enzyme to produce a distinctive colour. The colour strength is photometrically determined to advantageously give a quantitative indication of the number of antibody-antigen complexes which have been formed. This in turn gives an indication of the number of specific antigens present per unit volume of sample fluid.

Another advantage of ELISA procedures is that they do not suffer from the storage and disposal problems associated with radioimmunoassays.

Although it is common to look for specific antigens in a sample, it is also possible to look for specific antibodies which are produced by the body in response to an infection. In such cases, the detection of a large number of specific antibodies in a sample will indicate that a large number of corresponding antigens are also present. For example, a Rubella infection will result in the production by the body of a large number of antibodies to Rubella antigenic material. The detection of these antibodies in large numbers would indicate that the patient has been exposed to Rubella antigenic material.

Although other different testing procedures are available, ELISA remains one of the most commonly used because it is relatively inexpensive, has a high throughput and has good performance. There is also widespread availability of consumables and instrumentation required for the process.

Early known ELISA systems were run manually, and samples and dispensing reagents were transferred manually using pipettes. Sample containers were washed under a tap and the results were measured visually. However, as can be appreciated, manually operated systems suffered from a number of problems, including variable results with a limited dynamic range. The technician was also unduly exposed to potentially biohazardous material.

In recent years systems have been developed which automate many of the steps (or "phases") involved in the ELISA procedures such as sample distribution, dilution, incubation, washing, enzyme conjugate addition, reagent addition, reaction stopping and the analysis of results.

Automated immunoassay apparatus for carrying out ELISA procedures are widely used in clinical laboratories of e.g. pharmaceutical companies, hospitals and universities for in-vitro diagnostic applications such as testing for diseases and infection, and for assisting in the production of new vaccines and drugs.

Automated ELISA systems use a standard sample vessel known as a microplate which can be easily stored and which may be used with a variety of biological specimens. Microplates manufactured by the Applicants are sold under the name "MICROTITER" (RTM). However, the ELISA system described in the present application is designed to be an open system thereby allowing other manufacturers' microplates and other consumables to be used.

Microplates have been commercially available since the 1960s and consist of a reusable plate made from e.g. polystyrene, PVC, perspex or lucite and measuring approximately 5 inches (12.7 cm) in length, 3½ inches (8.9 cm) in width, and ¾ inches (1.9 cm) in depth.

Microplates made from polystyrene are particularly preferred on account of polystyrene's enhanced optical clarity which assists visual interpretation of the results of any reaction. Polystyrene microplates are also compact, lightweight, and easily washable.

Each microplate has 96 wells or indentations (also commonly known as "microwells") which are symmetrically arranged in an 8 x 12 array. Each microwell of a microplate will normally contain a sample from a different patient. The microwells are sometimes also referred to as the "solid phase" since they are considered to be the starting point upon which the rest of the testing procedures are based.

Microwells typically have a maximum volume capacity of approximately 350µl, but normally only 10-100µl of fluid is dispensed into a microwell.

Microplates having a flat-bottomed well geometry are widely accepted for bacteriology and other microbiology applications including tissue culture growth analysis and antibiotic sensitivity testing. However, microplates having "U" and "V" shaped well bottom geometries are also known and are used in complement fixation analysis so as to accommodate agglutination applications. "U" and "V" shaped microwells are effective in reducing the sample and reagent volume requirements and they also help concentrate the reaction in the well bottom thereby aiding the subsequent interpretation of results.

Flexible microplates made from polyvinyl chloride (PVC) are used in radioimmunoassays. These microplates are produced in the standard 96-well format, but individual wells can be removed with scissors for radioisotopic measurement using a single well gamma counter.

Other configurations of microplates exist including deep well microplates which have well capacities of 1200µl and are typically 1¾ inches (4.5 cm) in depth. Deep well microplates normally have the same length, width and well arrangement as a standard microplate.

A number of different variations of the ELISA technology are commercially available. However, all require that fluid samples e.g. blood, serum, urine etc. are aspirated from a sample tube and are then dispensed into a microwell of a microplate.

ELISA kits are commercially available and these consist of microplates having microwells which have been coated by the manufacturer with a specific antibody (or antigen). For example, in the case of a Rubella diagnostic kit, the kit manufacturer will dispense Rubella antibodies which have been suspended in a fluid into the microwells of a microplate. The microplate is then incubated for a period of time, during which time the antibodies adhere to the walls of the microwells up to the fluid fill level (typically about half the maximum fluid capacity of the microwell). The microwells are then washed leaving a microplate having microwells whose walls are uniformly covered with Rubella antibodies up to the fluid fill level.

A testing laboratory will receive a number of sample tubes containing, for example, body fluid samples from a number of patients. A specified amount of fluid is then aspirated out of the sample tube using a pipette mechanism and is then dispensed into one or more microwells of a microplate which has been previously prepared by the manufacturer as discussed above. If it is desired to test a patient for a number of different diseases then fluid from a patient may be dispensed into a number of different microplates. Each microplate can then be tested for the presence of a different disease.

The pipette mechanism used to aspirate and dispense fluid samples uses disposable tips which are ejected after being used so as to prevent cross-contamination of patients' samples.

Once the desired number of patients' samples have been dispensed into a microplate, the microplate is then placed in an incubator which speeds up the process of binding or antigen uptake (if applicable). Preferred incubation temperatures and incubation times are specified by the testing kit manufacturer. Incubation temperatures at around body temperature (37°C) are common, but different incubation temperatures may be used. The maximum incubation temperature is normally around 55°C. The incubation process usually lasts around half an hour, although incubation times of up to a few hours may sometimes be necessary.

After incubation the microplate is transferred from the incubator to a washer unit where all the microwells are thoroughly washed. Washing involves repeatedly filling the microwells with an inert fluid/detergent mixture ("wash buffer" solution). The fluid/detergent mixture is then aspirated out of all the microwells. Typically, five fill/aspirate cycles per microwell are required in order to wash sufficiently the microwells. The washing process is usually achieved by filling and aspirating through a manifold thereby allowing whole columns or rows of microwells to be filled/aspirated at the same time.

The wash fluid is usually supplied by the kit manufacturer and is intended to wash the microplate without damaging any antigen-antibody complexes which have been formed during the incubation phase. The washing phase is intended to remove any unbound proteins that would otherwise interfere with the subsequent analytical processes whilst leaving the antigen-antibody complexes intact.

Washing typically lasts around 5 minutes and can take place independently of other steps which might be required on other microplates. Failure to wash the microplate after sample incubation would de-sensitise the process as the fluid content of the sample needs to be removed for subsequent reagent additions to take place.

At the end of the washing phase the microwells are left empty apart from any antigen-antibody complexes that have formed. At this stage there is no visible difference between a negative and a positive sample.

At this stage the ELISA procedure has successfully emulated the immune system by capturing antigens suspended in a sample in-vitro. Antibodies which have been coated to the walls of the microplate during manufacture have, in the case of a positive sample, bonded to antigens present in the patient's sample.

The next stage in the ELISA procedures is to add an enzyme conjugate to the microwell that will attach or bind to the antigen part, but not to the antibody part, of any antigen-antibody complex which has been formed. Therefore, in the case of a negative sample where no antigen-antibody complexes have been formed and hence there is no antigenic material left in the microwell, then there is nothing for the enzyme conjugate to bind on to.

Once enzyme conjugate has been added to a sample the microplate is then usually placed once again in an incubator in order to accelerate any binding of the enzyme to the antigen part of any antigen-antibody complex. This further incubation step normally takes around 30 minutes.

Once the enzyme conjugate has been added and the microplate has been left to incubate, the microplate is then removed from the incubator and is washed once more. This time the microplate is washed to remove any unbound enzyme conjugate material. This will therefore either leave antigen-antibody complexes together with bound enzyme conjugate (in the case of a positive sample) or just the factory bound antibody (in the case of a negative sample).

The next stage in the diagnostic process is to add a fixed volume of a reagent (also known as a "substrate") to each microwell and optionally return the microplate to the incubator a yet further time. Alternatively, the microplate may simply be left to incubate at ambient room temperature.

Reagents, upon contact with the enzyme conjugate bound to the antigen part of any antigen-antibody complexes which are present, break down giving off a distinctive colour which typically has a narrow band wavelength. The breakdown of the reagent and the subsequent colour development usually reaches saturation after about 30 minutes. Once colour development has been satisfactorily completed, the microplates may be washed again. If the microplate were not washed after enzyme conjugate had been added then this would allow the enzyme conjugate to mix freely with reagent which is added at the next stage. This would result in colour being produced for all samples regardless of the presence of antigen-antibody complexes.

Enzyme conjugates and reagents (substrates) must be carefully chosen. The same reagent is often used for multiple analytes, i.e. Rubella, Hepatitis, HIV etc., but the enzyme conjugate is usually unique to the target analyte.

The process of incubating, adding reagent and washing may be repeated a number of times and different reagent types, incubation temperatures and wash parameters may be used on subsequent cycles.

Once any colour development has reached saturation, the microplates are ready for interpretation. Acid is usually added to prevent further colour development and this also has the advantage of leaving the microplate stable for a number of hours after the reaction has been stopped. The acid used is normally common across kits.

After the reaction has been stopped, the microplates are then interpreted by transferring the microplates to an optical reader which photometrically measures the amount of colour in each microwell. Narrow-band light is projected through each microwell and the transmitted light is measured. This enables the amount of absorption to be quantified and a corresponding output signal is produced. Results may then be sent to a host computer.

Alternatively, luminescent or fluorescent effects may be used, in which case light emission rather than light absorption is measured and quantified.

Controls and standards are also typically supplied by the kit manufacturer together with indications of expected results.

Controls are generally supplied with a qualitative kit such as Hepatitis testing and are used for quality control and to provide a relative cut-off. A few negative controls and normally one positive control are provided with the kit and are expected to give results within a range previously determined by the kit manufacturer. Following the reading of the microplate after substrate development, the results of the controls are checked. The positive control is checked to see if it has been reported as a positive result and the negative controls are checked to see if they are below a certain value. Results from controls that are within the manufacturers published acceptance criteria indicate that the kit and the testing process have worked correctly. The controls also provide a relative cut-off. For example, if the highest negative control is reported with a value of 0.5 then the kit instructions might indicate that any result above 0.5 should be expressed as a positive result. As the controls have been run on the same microplate as the samples being tested, this method provides a relative cut-off which compensates for any influencing factors associated with the process.

Standards are provided in order to give an expected result. They are usually used to build a standard curve for assays that require a quantitative result. For example, six standards having a different known concentration of analyte may be provided. By plotting on a graph the measured result (e.g. colour intensity) for each standard on the Y axis against the known concentration on the X axis, a curve of measured result versus concentration can be drawn up. This enables an unknown sample (which is usually processed on the same microplate) to be correlated against the curve so that the measured result can be expressed as a concentration.

Known automated immunoassay systems use a pipette mounted on a first arm to aspirate and dispense fluid. A second separate arm is then used to move microplates from one process stage (e.g. incubation, washing etc.) to another. Such a system requires the provision of multiple drive mechanisms - one to move the pipette around and another to move the microplates around. This results in a relatively complex, large and hence correspondingly expensive system.

It is therefore desired to provide an improved system.

According to a first aspect of the invention there is provided an automated immunoassay apparatus as claimed in claim 1.

The integral clamp provided as part of the pipette mechanism allows items such as a microplate to be moved from one discrete processing station, e.g. an incubator, washer or reader, to another, avoiding the need to duplicate such functions in hardware by e.g. having to provide a separate transporting arm. As well as picking up other items, the pick-up means is also used to pick up and preferably also eject a diposable tip used for aspirating and/or dispensing a fluid sample or reagent.

Preferably, the integral clamp is suitable for picking up and ejecting a disposable tip.

The clamp forms part of the pipette mechanism and may be referred to as a "plate clamp" or "pipette clamp". The pipette clamp has been particularly designed to pick-up a microplate, preferably carried by a plate holder, from a pipetting area and to move it to a desired module such as an incubator for a period of time dictated by a pre-programmed software parameter.

Preferably, the clamp comprises a collar which is generally axially movable. The clamp may comprise a generally fixed first part and a generally movable second part. Alternatively, the clamp may comprise two parts which are generally movable in opposed directions to pinch or otherwise clamp an item held therebetween. A portion of the clamp may be spring loaded or otherwise resilient.

By providing a clamp on the pipette mechanism it is no longer necessary to provide a second robotic arm for plate transport as is conventionally provided. A more compact, simpler and less expensive system can therefore be provided.

Preferably, the pipette mechanism is designed to engage with a disposable tip e.g. a sample or reagent tip and the mechanism is designed to pick up one or more items such as a microplate, a plate holder, one or more sample tubes, a sample rack, a reagent container, a reagent rack, a control container, a control rack, a tip rack, and similar consumable items.

Preferably, the integral clamp engages in use with a slot or recess provided in a plate holder pickup block connected or otherwise attached to a plate holder. The plate holder may carry, in use, a microplate or similar item.

Preferably, the integral clamp and/or the plate holder pickup block further comprise anti-rotation means for preventing the plate holder from rotating and to help keep it as steady as possible.

Preferably, the pipette mechanism has a sprung loaded cone portion for engagement with a disposable tip.

According to another aspect of the present invention, there is provided an Enzyme Linked ImmunoSorbent Assay system as claimed in claim 13.

According to a further aspect of the present invention, there is provided apparatus for diagnosing biological, chemical or biochemical products as claimed in claim 14.

According to a yet further aspect of the present invention, there is provided an Enzyme Linked ImmunoSorbent Assay apparatus as claimed in claim 15.

Various embodiments of the present invention together with other arrangement given for illustration purposes only will now be described, by way of example, and with reference to the accompanying drawings in which:
Fig. 1 shows an automated sample diagnostic or immunoassay system;
Fig. 2 shows a pipette mechanism together with an integral clamp according to an embodiment of the present invention;
Fig. 3 shows in greater detail the pipette mechanism and integral clamp together with a plate holder which is to be picked up by the clamp;
Fig. 4 shows a retractable drawer in a substantially fully retracted position; and
Fig. 5 shows a retractable drawer in a substantially fully extended position.

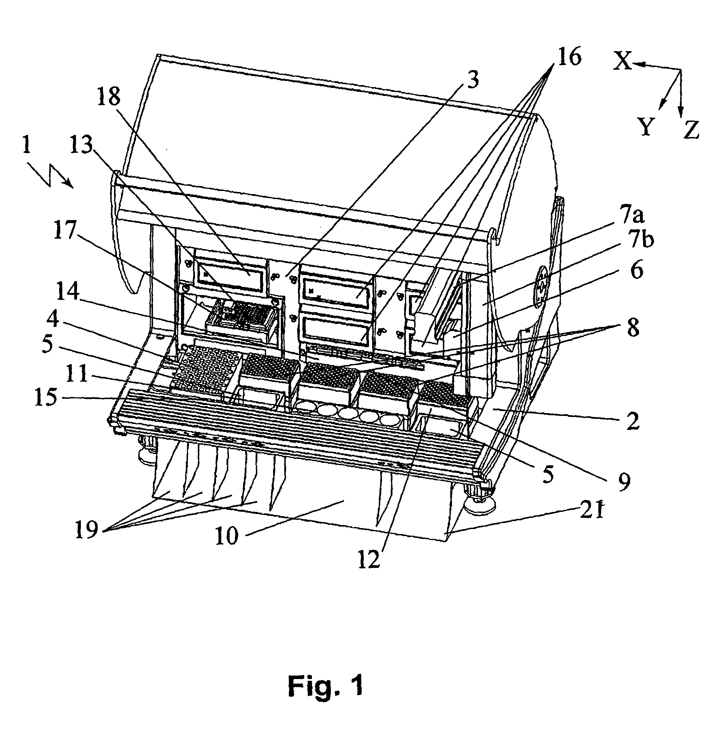

With reference to the drawings, Fig. 1 shows an automated sample diagnostic system or immunoassay apparatus 1 according to a preferred embodiment. The automated immunoassay apparatus 1 may be considered to comprise a "horizontal work area" 2 in which the various components and consumables necessary for carrying out the diagnostic tests are arranged. A number of self-contained modules 16-18 are arranged in what may be referred to as a "vertical work plane" 3 above the horizontal work area 2.

In the embodiment shown in Fig. 1, four incubator modules 16 are provided together with one washer module 17 and one reader or interpretation module 18. However, it will be appreciated that differing numbers of incubator or reader modules 16;18 may be provided and the positions of these modules 16;18 can be interchanged or otherwise varied. The washer module 17 may use up to four different wash fluids which are supplied by wash fluid containers (not shown) positioned in washer fluid container recesses 19 under the horizontal work area 2.

Patients' samples which are to be tested are provided in sample tubes (e.g. test tubes) which are loaded into one or more sample racks 4. Each sample rack 4 can carry ninety-six sample tubes arranged in an 8 x 12 array in a similar manner to the arrangement of the microwells of a microplate 13. There is therefore normally a one to one correspondence between the samples in a sample rack 4 and the microwells of a microplate 13.

It is often necessary to dilute the samples prior to processing and analysing them. Sample dilution may be carried out using a diluent loaded in a bottle in reagent rack 11 and one or more deep well dilution plates (not shown) located at deep well dilution plate positions 5. The diluent is transferred from the bottle to a deep well plate using a pipette mechanism 6.

The pipette mechanism 6 uses disposable tips to prevent cross-contamination of samples and contamination of subsequent processing steps. 300µl sample tips 9 are used for sample distribution and larger 1.3ml reagent tips are used for reagent dispensing. Smaller tips are used for sample distribution since precise and accurate pipetting at low volumes (e.g. 10µl) is required. Larger tips are not used for low volume sample pipetting since the amount of air present in the large tip above the sample could adversely influence pipetting precision. However, reagent dispensing is less critical and therefore a larger reagent tip can be used which allows a greater amount of reagent to be aspirated at any one time. This enables reagent to be dispensed into a large number of microwells before it is necessary to re-aspirate reagent.

Reagents, which are supplied in bottles, are preferably stored in reagent rack 11.

Disposable sample tips 9 and reagent tips are preferably stored in one or more tip racks 8;12. Preferably, each tip rack 8;12 has a capacity of 108 tips. In the embodiment shown in Fig. 1 four tip racks 8,12 are shown arranged towards the rear of the horizontal work area 2. Three of the tip racks 8 contain sample tips 9, and one of the tip racks 12 contains reagent tips. More sample tips 9 than reagent tips are usually provided due to a greater consumption rate of the former in normal operation.

Samples are transferred from sample tubes in sample rack 4 to a deep well dilution plate (not shown) located at a deep well dilution plate position 5 (if sample dilution is necessary) using the pipette mechanism 6 together with disposable sample tips 9. The diluted samples are then transferred from the deep well dilution plate to a microplate 13 preferably loaded on a retractable drawer 14 which is shown in a closed or retracted position in Fig. 1. The retractable drawer 14 is a space saving device which enables a smaller horizontal work area 2 to be utilised compared with other known apparatus.

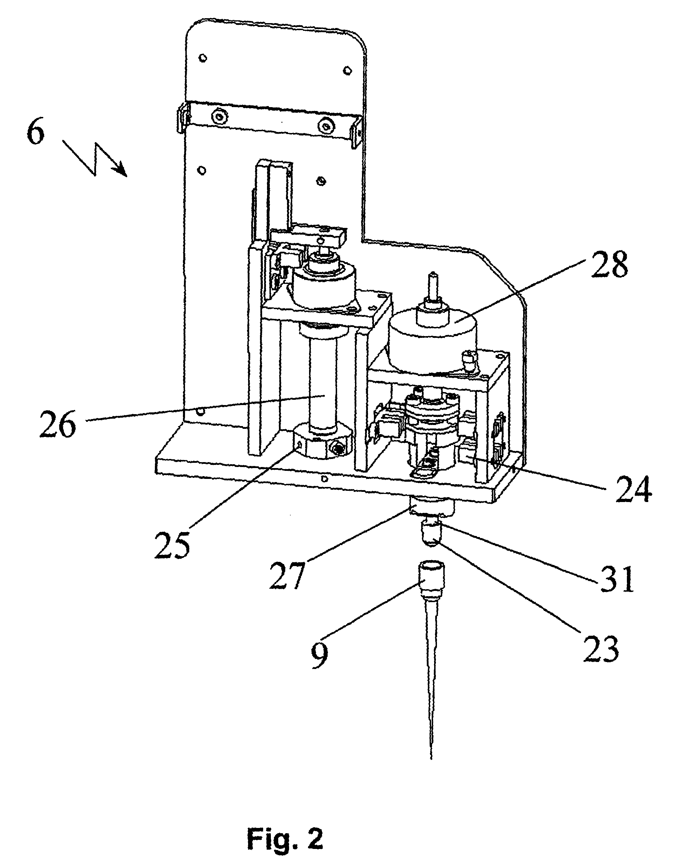

The pipette mechanism 6 is shown in greater detail in Fig. 2. The pipette mechanism 6 has a spring loaded cone portion or tip pickup assembly 23 mounted below a clamp spigot 31. The cone portion 23 is engageable with both disposable sample 9 and reagent tips.

The pipette mechanism 6 is mounted to or otherwise provided on a robotic or otherwise movable arm 7a,7b which enables the pipette mechanism 6 to be moved to any desired position within the volume bounded by the horizontal work area 2 and the vertical work plane 3 e.g. adjacent a tip rack 8;12 or one of the various modules 16-18.

The movable arm 7a,7b may comprise a horizontal component 7a which allows a vertical component 7b to move in a first horizontal (y) direction (see Fig. 1) and a vertical component 7b which allows the pipette mechanism 6 and pick-up means to move in a vertical (z) direction. The movable arm 7a,7b is translatable in an orthogonal second horizontal (x) direction. The pipette mechanism 6 and pick-up means is therefore able to move over the full extent of the horizontal work area 2 and can also be raised or lowered over the full extent of the vertical work plane 3 as desired.

In order to pick up a disposable sample tip 9 or reagent tip from a tip rack 8;12, the pipette mechanism 6 is positioned over the tip and is lowered towards it. The sprung loaded cone portion 23 will engage the tip and will make firm contact with it. Once the tip has been loaded on to the taper of the cone portion 23, the pipette mechanism 6 can then be raised or otherwise withdrawn and moved to a desired position with the tip attached in readiness for aspirating and/or dispensing fluid.

If it is desired to aspirate fluid from a sample vessel, then the pipette mechanism 6 can be moved into position over the sample in the horizontal work area 2 by the moveable arm 7a,7b and driven towards the sample. Preferably at the same time as the pipette mechanism 6 is being lowered towards the sample, the pipette mechanism 6 is activated so as to aspirate whilst it is being lowered towards the fluid sample. A fluid pressure sensor (not shown) connected to pressure outlet 25 may be provided to detect whether or not any fluid is being aspirated and the pipette mechanism 6 can be controlled accordingly. Fluid may be aspirated into the tip using a positive air displacement syringe assembly 26 which is connected to the internal bore of the pipette mechanism 6 by a tube (not shown).

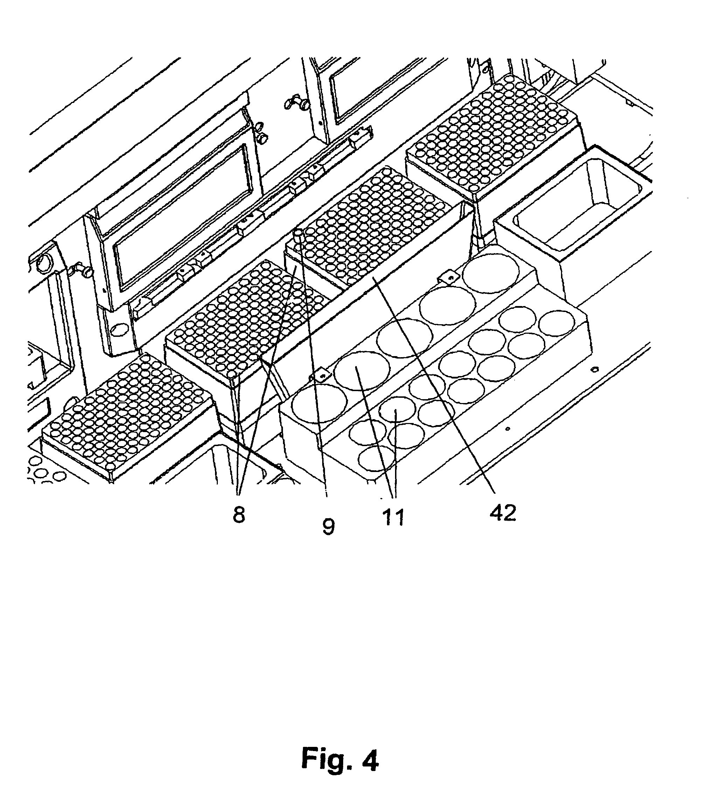

A movable clamp collar 27 is provided above the clamp spigot 31 and is downwardly slidable thereupon towards the cone portion 23. Once a tip is finished with or is otherwise spent, it can be automatically ejected from the pipette mechanism 6. Tip ejection is performed by activating a tip ejection motor 28 which drives the clamp collar 27 in a downwards direction, sliding over the clamp spigot 31 to push the tip off the end of the cone portion 23. Spent tips are preferably ejected into a waste tip container (not shown) via a tip chute 42 (see Fig. 4). In one embodiment the tip chute 42 may be located between the reagent rack(s) 11 and the tip racks 8,12. The waste tip container is preferably located below the tip chute 42 in a waste tip container recess 10. A waste fluid container (not shown) is also preferably provided in a waste fluid container recess 21 below the horizontal work area 2.

A tip sensor 24 monitors, preferably continuously, whether or not a disposable tip is attached or loaded to the pipette mechanism 6. If a tip is present, then the pipette mechanism 6 may be instructed by control means (not shown) to proceed to aspirate/dispense fluid as desired. However, if the tip sensor 24 determines that a tip is not present, then the pipette mechanism 6 may be instructed to commence a tip loading operation.

Controls and standards, loaded in bottles held in a controls rack 15, preferably located adjacent to the reagent rack 11, provide calibration values for predetermined tests and can be dispensed into microplates 13 in the same manner as patient samples.

Microplates 13 and other desired items are transferred from one part of the apparatus 1 to another using the clamp device 23,27,31 which preferably forms part of the pipette mechanism 6.

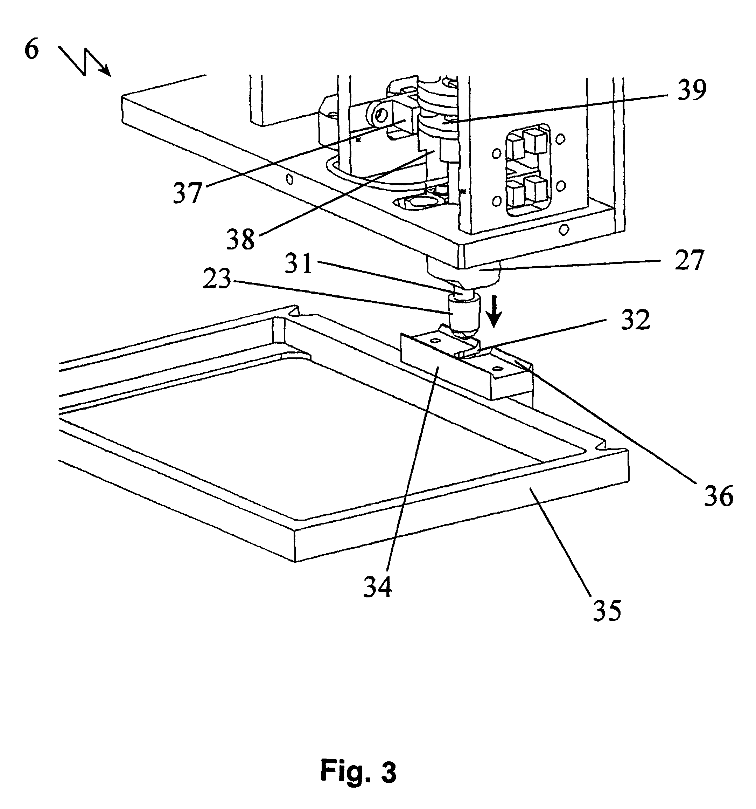

Fig. 3 shows in greater detail the pipette mechanism 6 and the integral clamp device 23,27,31 which is used for transporting microplates and other consumables. The pipette mechanism 6 is moved to a plate pick-up position wherein the clamp spigot 31 together-with the upper downwardly movable clamp collar 27 and the lower fixed cone portion 23 (which is generally, preferably substantially, resistant to downward movement) engage with a cooperating slot or recess 32 provided in a plate holder pick-up block 34 attached to a plate holder 35. The plate holder 35 consists of a frame which can support a microplate 13. Other items may also be moved when fitted with a pick-up block 34 such as one or more sample tubes in a sample rack 4, a reagent container in a reagent rack 11, a tip rack 8;12 or another similar item. Once in the correct position, the movable clamp collar 27 is driven downwards so as to firmly grip or otherwise secure the pick-up block 34.

The movable clamp collar 27 may have one or more tapered or angled faces which preferably engage with one or more correspondingly tapered or angled sides 36 of the pick-up block 34 so as to ensure that any item carried is kept substantially horizontal, generally steady and preferably also at least generally free from rotation whilst it is being transported. A sensor 37 may be provided to check that the item fitted with the pick-up block 34 has been picked up correctly. Rotational movement of the pick-up block 34 is kept to a minimum by a slot 38 which restricts the rotational movement of the cylindrical plate clamping assembly 39 about its vertical axis.

Fig. 4 shows in greater detail the work area and the components that occupy the work area under the retractable drawer 14 (shown in a retracted position). In one arrangement, one or more of the tip racks 8 are disposed below the retractable drawer 14 so as to be exposed only when the retractable drawer 14 is in a fully, or generally closed state. A tip chute 42 may also be exposed. The tip chute 42 may be provided as a means for funnelling used or spent tips into a tip waste container (not shown). One or more reagent racks 11 may also be exposed when the retractable drawer 14 is retracted.

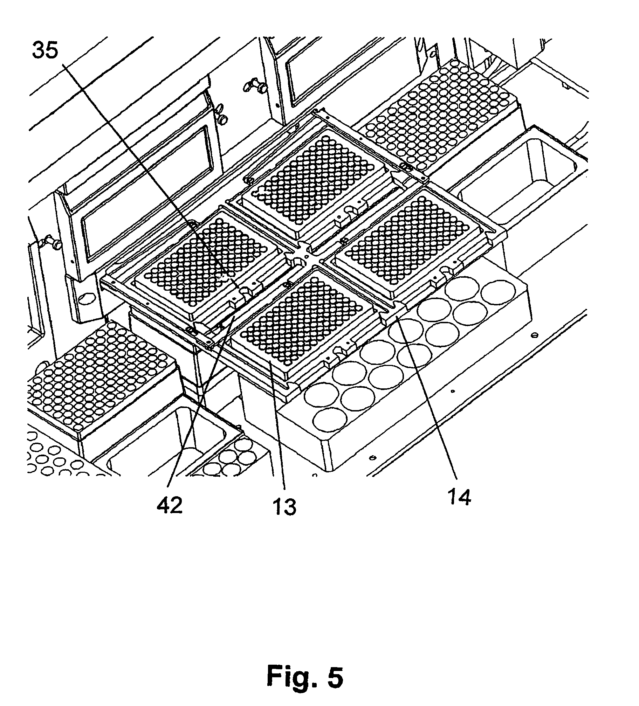

Fig. 5 shows the retractable drawer 14 ejected so as to cover at least part of the horizontal work area 2. In the arrangement shown, two tip racks 8 and one reagent rack 11 are covered when the retractable drawer 14 is extended, and four microtiter plate holders 35 are shown disposed on the retractable drawer 14. Microplates 13 may be clipped or otherwise secured in/to the plate holders 35. The tip waste chute 42 remains accessible when the retractable drawer 14 is extended by the provision of an aperture in the drawer plate carrier.

## Claims

1. An automated immunoassay apparatus (1) comprising:
a movable arm (7a, 7b);
a pipette mechanism (6) for aspirating and/or dispensing fluid, said pipette mechanism (6) being connected to said movable arm (7a, 7b);
**characterised in that**:
said pipette mechanism further comprises an integral clamp (23,27,31) for picking up and transporting one or more items other than a disposable tip (9).

2. An automated immunoassay apparatus as claimed in claim 1, wherein said clamp (23,27,31) comprises a collar (27).

3. An automated immunoassay apparatus as claimed in claim 2, wherein said collar (27) is movable in a first axial direction.

4. An automated immunoassay apparatus as claimed in claim 1, 2 or 3, wherein said clamp (23,27,31) comprises a portion (23) for engagement with a disposable sample tip (9) or a reagent tip.

5. An automated immunoassay apparatus as claimed in claim 4, wherein said portion (23), in use, substantially resists movement in said first axial direction.

6. An automated immunoassay apparatus as claimed in claim 4 or 5, wherein said portion (23) is spring loaded.

7. An automated inmunoassay apparatus as claimed in claim 6, wherein said portion (23) may move in a direction opposed to said first axial direction.

8. An automated immunoassay apparatus as claimed in any of claims 4-7, wherein said portion (23) co-operates with said collar (27) to clamp a pickup block (34) or other article disposed between said collar (27) and said portion (23)

9. An automated immunoassay apparatus as claimed in any preceding claim, wherein said one or more items are selected from a group comprising: a microplate (13), a plate holder (35), one or more sample tubes, a sample rack (4), a reagent container, a reagent rack (11), a control container, a control rack (15), and a tip rack (8;12).

10. An automated immunoassay apparatus as claimed in any preceding claim, further comprising a pickup block (34) and wherein said clamp (23,27,31) engages in use with a slot or recess (32) provided in said pickup block (34).

11. An automated immunoassay apparatus as claimed in claim 10, wherein said pickup block (34) is connected to a holder (35) suitable for carrying a microplate (13).

12. An automated immunoassay apparatus as claimed in claim 10 or 11, wherein said clamp (23,27,31) and/or said pickup block (34) further comprise anti-rotation means (27,36) for substantially preventing said pickup block (34) and any item attached thereto from rotating.

13. An Enzyme Linked ImmunoSorbent Assay system comprising automated immunoassay apparatus as claimed in any preceding claim.

14. A method of automatically performing immunoassay procedures comprising:
providing a movable arm (7a, 7b);
aspirating and/or dispensing fluid using a pipette mechanism (6), said pipette mechanism (6) being connected to said movable arm (7a,7b);
**characterised in that** said method further comprises the step:
picking up and transporting one or more items other than a disposable tip (9) using said pipette mechanism (6) having an integral clamp (23,27,31).

15. A method as claimed in claim 14, wherein said method is used for carrying out Enzyme Linked ImniunoSorbent Assay procedures.

## Patentansprüche

1. Automatisierte Immunprüfvorrichtung (1) mit:
einem bewegbaren Arm (7a, 7b),
einem Pipettenmechanismus (6) zum Absaugen und/oder Abgeben von Fluid, wobei der Pipettenmechanismus (6) mit dem bewegbaren Arm (7a, 7b) verbunden ist,
**dadurch gekennzeichnet, daß**
der Pipettenmechanismus weiterhin eine integrale Klammer bzw. Klemme (23, 27, 31) zum Aufnehmen und Transportieren eines oder mehrerer Gegenstände außer einer Wegwerfspitze (9) umfaßt.

2. Automatisierte Immunprüfvorrichtung nach Anspruch 1, bei der die Klemme (23, 27, 31) einen Bund bzw. eine Einfassung aufweist.

3. Automatisierte Immunprüfvorrichtung nach Anspruch 2, bei der der Bund (27) in einer ersten axialen Richtung bewegbar ist.

4. Automatisierte Immunprüfvorrichtung nach Anspruch 1, 2 oder 3 bei der die Klemme (23, 27, 31) einen Abschnitt (23) zum Eingreifen in eine Einwegprobespitze (9) oder Reagenzspitze aufweist.

5. Automatisierte Immunprüfvorrichtung nach Anspruch 4, bei der der Abschnitt (23) bei Gebrauch im wesentlichen einer Bewegung in die erste axiale Richtung widersteht bzw. dieser entgegensteht.

6. Automatisierte Immunprüfvorrichtung nach Anspruch 4 oder 5, bei der der Abschnitt (23) federbelastet ist.

7. Automatisierte Immunprüfvorrichtung nach Anspruch 6, bei der der Abschnitt (23) sich in eine Richtung entgegengesetzt der ersten axialen Richtung bewegen kann.

8. Automatisierte Immunprüfvorrichtung nach einem der Ansprüche 4 bis 7, bei der der Abschnitt (23) mit dem Bund (27) zusammenwirkt, um einen Aufnahmeblock (34) oder einen anderen Gegenstand, der zwischen dem Bund (27) und dem Abschnitt (23) angeordnet ist, festzuklemmen.

9. Automatisierte Immunprüfvorrichtung nach einem der vorstehenden Ansprüche, bei der der eine oder die mehreren Gegenstände aus einer Gruppe ausgewählt sind, die eine Mikroplatte (13), einen Plattenhalter (35) eine oder mehrere Proberöhren, ein Probegestell (4), einen Reagenzcontainer, ein Reagenzgestell (11), einen Kontrollcontainer, ein Kontrollgestell (15) und ein Spitzengestell (8, 12) umfaßt.

10. Automatisierte Immunprüfvorrichtung nach einem der vorstehenden Ansprüche, die weiterhin einen Aufnahmeblock (34) aufweist und bei der die Klemme (23, 27, 31) bei Gebrauch in einen Schlitz oder eine Aushöhlung bzw. Aussparung (32) eingreift, die in dem Aufnahmeblock (34) vorgesehen ist.

11. Automatisierte Immunprüfvorrichtung nach Anspruch 10, bei der der Aufnahmeblock (34) mit einem Halter (35) verbunden ist, der zum Tragen einer Mikroplatte (13) geeignet ist.

12. Automatisierte Immunprüfvorrichtung nach Anspruch 10 oder 11, bei der die Klemme (23, 27, 31) und/oder der Aufnahmeblock (34) weiterhin ein Antirotationsmittel (27, 36) aufweist, um im wesentlichen den Aufnahmeblock (34) und jeden damit verbundenen Gegenstand von einem Rotieren abzuhalten.

13. Enzym-Immuntestsystem mit einer automatisierten Immunprüfvorrichtung nach einem der vorstehenden Ansprüche.

14. Verfahren zum automatischen durchführen von Immunprüfvorgängen mit den Verfahrensschritten:
Bereitstellen eines bewegbaren Arms (7a, 7b),
Absaugen und/oder Abgeben von Fluid unter Verwendung eines Pipettenmechanismus (6) wobei der Pipettenmechanismus mit dem bewegbaren Arm (7a, 7b) verbunden ist,
**dadurch gekennzeichnet, daß** das Verfahren weiterhin folgende Schritte umfaßt:
Aufnehmen und Transportieren eines oder mehrerer Gegenstände außer einer Einwegspitze (9) unter Verwendung des Pipettenmechanismus (6) mit einer integralen Klemme (23, 27, 31).

15. Verfahren nach Anspruch 14, bei dem das Verfahren zum Ausführen von Enzym-Immuntest-Prozeduren verwendet wird.

## Revendications

1. Appareil de dosage immunologique automatisé (1), comprenant :
un bras mobile (7a, 7b) ;
un mécanisme de pipette (6) pour aspirer et/ou distribuer un fluide, le dit mécanisme de pipette (6) étant connecté au dit bras mobile (7a, 7b) ;
**caractérisé en ce que** :
le dit mécanisme de pipette comprend en outre une pince solidaire (23, 27, 31) pour saisir et transporter un ou plusieurs éléments autres qu'un embout jetable (9).

2. Appareil de dosage immunologique automatisé selon la revendication 1, dans lequel la dite pince (22, 27, 31) comprend un collier (27).

3. Appareil de dosage immunologique automatisé selon la revendication 2, dans lequel le dit collier (27) est déplaçable dans une première direction axiale.

4. Appareil de dosage immunologique automatisé selon la revendication 1, 2 ou 3, dans lequel la dite pince (23, 27, 31) comprend une portion (23) pour venir en prise avec un embout d'échantillon jetable (9) ou un embout de réactif.

5. Appareil de dosage immunologique automatisé selon la revendication 4, dans lequel la dite portion (23), en utilisation, résiste substantiellement à un mouvement dans la dite première direction axiale.

6. Appareil de dosage immunologique automatisé selon la revendication 4 ou 5, dans lequel la dite portion (23) est chargée élastiquement.

7. Appareil de dosage immunologique automatisé selon la revendication 6, dans lequel la dite portion (23) peut se déplacer dans une direction opposée à la dite première direction axiale.

8. Appareil de dosage immunologique automatisé selon une quelconque des revendications 4 à 7, dans lequel la dite portion (23) coopère avec le dit collier (27) pour pincer un bloc de prise (34) ou un autre article disposé entre le dit collier (27) et la dite portion (23).

9. Appareil de dosage immunologique automatisé selon une quelconque des revendications précédentes, dans lequel les dits un ou plusieurs éléments sont choisis dans un groupe comprenant : une microplaque (13), un porte-plaque (35), un ou plusieurs tubes à échantillon, un râtelier d'échantillons (4), un récipient de réactif, un râtelier de réactifs (11), un récipient de contrôle, un râtelier de contrôle (15) et un râtelier d'embouts (8 ; 12) ;

10. Appareil de dosage immunologique automatisé selon une quelconque des revendications précédentes, comprenant en outre un bloc de prise (34), et dans lequel la dite pince (23, 27, 31) vient en prise, en utilisation, avec une fente ou un évidement (32) prévu dans le dit bloc de prise (34).

11. Appareil de dosage immunologique automatisé selon la revendication 10, dans lequel le dit bloc de prise (34) est connecté à une monture (35) prévus pour porter une microplaque (13).

12. Appareil de dosage immunologique automatisé selon la revendication 10 ou 11, dans lequel la dite pince (23, 27, 31) et/ou le dit bloc de prise (34) comprennent en outre des moyens anti-rotation (27, 36) pour empêcher sensiblement le dit bloc de prise (34) et tout élément attaché à celui-ci de tourner.

13. Système d'analyse d'immunosorption à liaison d'enzyme, comprenant un appareil de dosage immunologique automatisé selon une quelconque des revendications précédentes.

14. Procédé pour exécuter automatiquement des procédures de dosage immunologique, comprenant :
la réalisation d'un bras mobile (7a, 7b) ;
l'aspiration et/ou la distribution de fluide au moyen d'un mécanisme de pipette (6), le dit mécanisme de pipette (6) étant connecté au dit bras mobile (7a, 7b) ;
**caractérisé en ce que** le dit procédé comprend en outre l'étape de :
prise et transport d'un ou plusieurs éléments autres qu'un embout jetable (9) au moyen du dit mécanisme de pipette (6) comportant une pince solidaire (23, 27, 31).

15. Procédé selon la revendication 14, dans lequel le dit procédé est employé pour exécuter des procédures d'analyse d'immunosorption à liaison d'enzyme.
